# EUROPEAN PATENT APPLICATION

(11) **EP 1 411 048 A1**
(43) Date of publication of application: **21.04.2004**
(21) Application number: 03021622.0
(22) Date of filing: 25.09.2003
(51) Int. Cl.: C07D 239/30

(54) **Preparation of 2-bromo-pyrimidines and 2-iodo-pyrimidines**

(30) Priority: 17.10.2002 EP 02023248
(71) Applicant: MERCK PATENT GmbH, 64293 Darmstadt (DE)
(72) Inventor: Farrand, Louise Diane, Dr., Blandford Forum Dorset DT11 9ED (GB); Adlem, Kevin, Bournemouth Dorset BH8 9LS (GB)

(57) **Abstract**

The present invention is directed to a method for preparation of 2-bromo-substituted pyrimidines, in particular 2-bromo-5-alkylpyrimidines, by reaction of 2-chloro-substituted pyrimidines with hydrogen bromide or hydrogen iodide dissolved in an acid.

## Description

The present invention is directed to a method for preparation of 2-bromo-substituted pyrimidines. In particular, it is directed to the synthesis of 2-bromo-5-alkylpyrimidines.

Pyrimidine derivatives are extremely important chemical compounds in a wide range of applications such as agrochemicals, pharmaceuticals, and liquid crystals. For assembling complex pyrimidine derivatives easy accessible and versatile pyrimidine building blocks are desired. For instance, formation of C-C single bonds via palladium catalyzed cross-coupling reactions such as the Suzuki type [N. Miyaura, A. Suzuki, *Chem. Rev.* **1995,** *95*, 2457-2483] is a very powerful synthetic approach for building up pyrimidine containing molecules. However, while 2-chloro-substituted pyrimidines like 2-chloro-5-alkylpyrimidines are readily available compounds, in most cases those cross-coupling reactions which employ 2-chloro-substituted pyrimidines as building blocks proceed poorly if at all. On the other hand, 2-bromo-substituted or 2-iodo-substituted pyrimidines that are expected to be more suitable reactants for the palladium catalyzed cross-coupling reactions are sometimes commercially and even synthetically unavailable. In other cases their syntheses are cumbersome and of low efficiency.

This is especially true for 2-bromo- and 2-iodo substituted compounds of general formula I: 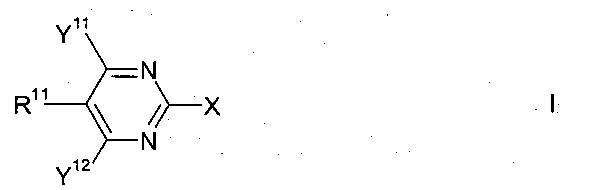 (in which X denotes Br or I and R¹¹, Y¹¹ and Y¹² are defined as below.)

In particular, there is no efficient synthesis known in the literature for preparing 2-bromo- and 2-iodo-substituted 5-alkyl-pyrimidines which compounds are of great importance for building up liquid crystals containing pyrimidine units.

C.M. Hudson et al., *Liquid Crystals*, **1999,** *26*, 241-250, describe the conversion of 5-(n-heptyl)-2-hydrazinopyrimidine (that is in turn available by reaction of a 2-chloro-substituted pyrimidine with the extremely toxic hydrazine hydrate) to 2-bromo-5-(n-heptyl)-pyrimidine with Br₂ in acetic acid and with cooling in a yield of only 31 %.

Therefore the present invention faces the problem of providing a clean and efficient preparation of 2-bromo-substituted or 2-iodo-substituted pyrimidines of general formula I.

This problem is solved by a method for preparation of a compound according to general formula I: 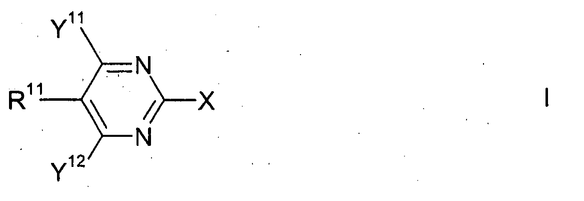 in which
- R¹¹: is hydrogen, alkyl, alkoxy, oxaalkyl, thioalkyl, cyclohexyl, aryl, aralkyl or heterocyclyl;
- X: is Br or I; and
- Y¹¹ and Y¹²: are, independently of each other, hydrogen, F, CN, alkyl, alkoxy, oxaalkyl, thioalkyl, cyclohexyl, aryl, aralkyl or heterocyclyl;
that is characterized in that a compound of general formula II: 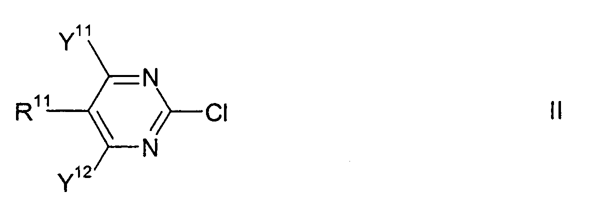 in which R¹¹, Y¹¹ and Y¹² are defined as in formula I above,
is reacted with a solution of HX in an acid, with X being Br or I.

The method according to the invention provides the desired compound of formula I in good to high yields. The reaction proceeds quickly and very cleanly without cooling and does not require the use of any highly toxic reagent. What is more, it starts with easily accessible starting material - 2-chloro-substituted pyrimidines of formula II which are commercially available (e.g. from Avocado Research, UK, Maybridge Chemical Company Ltd., UK, Apollo Scientific Ltd., UK, and Syntec GmbH, Germany) or can be easily synthesized by methods well known to those skilled in the art - and readily available reagents.

(In contrast, the use of conventional halogenating agents is not successful; for example, aqueous HBr or aqueous Hl yield at best a 1:1 mixture of starting material and the desired product of formula I which mixture cannot be separated by conventional techniques, and potassium iodide in acetone gives no product at all.)

Compounds of formula I available by use of the invention's method are suitable intermediates for the synthesis of complex pyrimidines. They are especially useful and advantageous over the 2-chloro-substituted analogue in transition metal catalyzed C-C coupling reactions such as the Suzuki type which allows the building up of elongated organic molecules containing a pyrimidine moiety and exhibiting mesogenic physical properties. Thus, the method according to the invention offers a simple way for manufacturing liquid crystals having a pyrimidine unit.

In the context of the present invention the term "alkyl" means straight-chain and branched saturated hydrocarbon radicals with 1 to 16 carbon atoms; the hydrocarbon radicals may be unsubstituted or substituted with one or more substituents being independently selected from the group consisting of F, Cl, Br, I, OH, SH, CN, amine, NO₂, cyclopropyl, cyclobutyl, cyclopentyl, cyclohexyl, and cycloheptyl. Furthermore, "alkyl" is also meant to comprise hydrocarbon radicals in which one or more of the CH₂ groups are such replaced by -CO-O- or -O-CO- that there are no adjacent oxygen atoms. Preferably, alkyl is a straight-chain or branched saturated hydrocarbon having 1, 2, 3, 4, 5, 6, 7 or 8 carbon atoms and being unsubstituted or mono- or poly-substituted with F. More preferably, alkyl is meant to be methyl, ethyl, n-propyl, i-propyl, n-butyl, i-butyl, t-butyl, n-pentyl, neopentyl, n-hexyl, n-heptyl, n-octyl. Most preferably, alkyl is a straight-chain hydrocarbon of up to 8 carbon atoms.

In the context of the present invention the term "alkoxy" means "O-alkyl" in which the oxygen atom is directly linked to the group or ring being substituted with alkoxy and alkyl is defined as above. In particular, "alkyl" in "O-alkyl" means methyl, ethyl, n-propyl, i-propyl, n-butyl, i-butyl, t-butyl, n-pentyl, neopentyl, n-hexyl, n-heptyl or n-octyl, whereby alkyl is optionally substituted with F. Most preferably, alkoxy is -OCH₃, -OC₂H₅, -O-i-C₃H₇, -O-n-C₄H₉, -O-t-C₄H₉, -OCF₃, -OCHF₂, -OCH₂F or -OCHFCHF₂.

In the context of the present invention the term "oxaalkyl" comprises alkyl moieties in which at least one non-terminal CH₂ group is replaced by O in such a way that there are no adjacent oxygen atoms. Preferably, oxaalkyl comprises straight-chain radicals of the formula CₙH₂ₙ₊₁-O-(CH₂)ₘ- in which n and m are independently of each other 1, 2, 3, 4, 5 or 6; especially n is 1 or 2 and m is an integer from 1 to 6.

In the context of the present invention the term "thioalkyl" comprises alkyl moieties in which at least one terminal or non-terminal CH₂ group is replaced by S (sulfur) in such a way that there are no adjacent sulfur atoms; it may also be designated as "S-alkyl". Preferably, thioalkyl comprises straight-chain radicals of the formula CₙH₂ₙ₊₁-S-(CH₂)ₘ- in which n is 1, 2, 3, 4, 5 or 6 and m is 0, 1, 2, 3, 4, 5 or 6; especially n is 1 or 2 and m is zero or an integer from 1 to 6.

In the context of the present invention the term "cycloalkyl" comprises alicyclic hydrocarbon radicals with 3 to 10 carbon atoms; the alicyclic hydrocarbon radicals may be unsubstituted or substituted with one or more substituents being independently selected from the group consisting of F, Cl, Br, I, OH, SH, CN, amine, NO₂, alkyl, alkoxy, oxaalkyl or thioalkyl, in which alkyl, alkoxy, oxaalkyl and thioalkyl are defined as above. Preferably, cycloalkyl includes cyclopropyl, cyclobutyl, cyclopentyl, cyclohexyl, and cycloheptyl all of which may be unsubstituted or substituted with an alkyl radical. More preferred, cycloalkyl includes cyclohexyl substituted in its 4-position with an alkyl radical.

In the context of the present invention the term "aryl" comprises aromatic hydrocarbon moieties; the aromatic hydrocarbon moieties may be unsubstituted or substituted with one or more substituents being independently selected from the group consisting of F, Cl, Br, I, OH, SH, CN, amine, NO₂, alkyl, alkoxy, oxaalkyl, thioalkyl, cycloalkyl, and heterocyclyl. Preferably, aryl includes unsubstituted phenyl or naphthyl or phenyl substituted with 1, 2 or 3 of the same or different groups being selected from F, alkyl, alkoxy, fluorinated alkoxy and cycloalkyl. When aryl is a substituted phenyl, it is preferred to have at least a substituent in 4-position of the phenyl.

In the context of the present invention the term "aralkyl" means an aryl being linked to the group or ring substituted via an alkyl radical. For instance, aralkyl means a phenyl-CH₂-CH₂- (phenethyl) moiety. It is preferred that aralkyl includes an aryl-ethyl moiety in which at least the 4-position of aryl is substituted and optionally one or two further positions of the aryl ring are substituted with any one of the substituents of "aryl" as described above.

In the context of the present invention the term "heterocyclyl" comprises saturated and unsaturated heterocyclic rings including heteroaromatics; the heterocyclyl moieties may be unsubstituted or substituted with one or more substituents being independently selected from the group consisting of F, Cl, Br, I, OH, SH, CN, amine, NO₂, alkyl, alkoxy, oxaalkyl, thioalkyl, cycloalkyl, aryl, and aralkyl. Preferably, heterocyclyl includes pyrrolidine, piperidine, piperazine, morpholinyl, and further pyrimidine moieties all of which may be substituted.

The halogenating agent of the present invention, HX in an acid, is readily available either as an instant solution or by introducing the gaseous HBr or HI, respectively, into the acid. The exact concentration and amount of HX used are not crucial for the outcome of the reaction. For example, when X = Br, a solution of 30 wt.% HBr in glacial acetic acid gives excellent yields. HX may be used in an equimolar amount or in an excess, e.g. in a 1.1-fold or 1.5-fold up to 3-fold or even 6-fold excess.

In one preferred embodiment of the invention X in formula I and in HX is Br providing 2-bromo-substituted pyrimidines of formula I in excellent yields.

Furthermore, it is preferred that both Y¹¹ and Y¹² in formulas I and II are hydrogen. Consequently, the method according to the invention then yields 2-bromo- or 2-iodo-5-substituted pyrimidines which are preferred for the synthesis of liquid crystals containing a pyrimidine ring by utilizing C-C-coupling reactions.

Moreover, it is preferred that R¹¹ in formulas I and II is alkyl or cycloalkyl. It is most preferred that R¹¹ in formulas I and II is a straight-chain alkyl radical.

The acid used as a solvent for the halogenating agent HX may be any inorganic or preferably organic acid suitable to dissolve the agent HX and, preferably, the pyrimidine of formula II in order to facilitate the conversion of the 2-chloro-substituted pyrimidine. In particular, the acid is a carboxylic acid. It is even more preferred that said carboxylic acid is non-aqueous and selected from the group consisting of formic acid, acetic acid, propionic acid, and trifluoroacetic acid. The solvent may also be a mixture of two or more of said acids. Most preferred the acid is glacial acetic acid.

The method according to the invention may be run at any temperature providing a sufficient conversion rate. It is preferred that the reaction is performed at a temperature between room temperature (about 20 °C) and the solvent's reflux temperature. It may also be useful to initiate the reaction in a reaction step (A) at a rather moderate temperature, e.g. between about 20 and about 30 or 35 °C, and to drive it to completion if necessary at elevated temperatures, preferably by refluxing the reaction mixture afterwards in a reaction step (B). The reaction time depends on the actual rate of the individual reaction. It may be useful to run the reaction within a period of 1 min up to 24 h, preferably within a period of 15 min up to 8 h, more preferably within a period of 30 min up to 2 h.

The reaction product of formula I prepared according to the invention's method may be isolated by usual work-up and purification with standard procedures well known to those skilled in the art. However, due to the high yields and the very clean reaction outcome, the product may also be used without further purification. For instance, the 2-bromo-alkylpyrimidines obtainable by use of the invention's method can be further reacted without purification with an aromatic boronic acid applying Suzuki reaction conditions in order to afford a liquid crystalline compound containing a pyrimidine moiety.

The following Examples are to illustrate the present invention but not meant to limit its scope of protection.

### Examples

The starting materials, reagents and solvents were purchased from Maybridge Chemical Company Ltd., UK (5-propyl-2-chloropyrimidine), Aldrich Chemical Company, US (HBr solution), Lancaster, UK (3,5-difluorophenylboronic acid; Pd catalyst), and VWR, UK.

For analytical purposes HPLC (Hewlett Packard 1090 with a Licrospher 100 column) and GC-MS (Agilent 5973 Detector and GC 6890) were used.

### Example 1

### Preparation of 5-propyl-2-bromopyrimidine

5-Propyl-2-chloropyrimidine (10 g) was stirred in hydrogen bromide dissolved in glacial acetic acid (60 ml; 30 wt.% HBr in the solvent) for 90 min at 30 °C, then under reflux for 15 min. The reaction showed 100 % conversion to the bromide by GC-MS. The solution was poured onto ice, extracted with diethyl ether and the organic phase was washed with sodium carbonate, water and then dried over sodium sulphate. Evaporation afforded a pale yellow liquid, 12.5 g (99 % purity by HPLC, GC-MS). GC-MS showed the mass ion (M⁺ 201).

### Example 2

### Suzuki cross-coupling of 5-propyl-2-bromopyrimidine with 3,5-difluorophenylboronic acid

A mixture of 5-propyl-2-bromopyrimidine (2.5 mmol), 3,5-difluorophenylboronic acid (2.5 mmol), sodium carbonate (4.5 mmol), toluene (3 ml), ethanol (2 ml), water (2 ml) and a catalytic amount of tetrakis(triphenylphosphine) palladium (0) was stirred under reflux over night. GC-MS of the reaction mixture showed complete conversion to the desired product, 2-(3,5-difluorophenyl)-5-propylpyrimidine.

## Claims

1. Method for preparation of a compound of general formula I 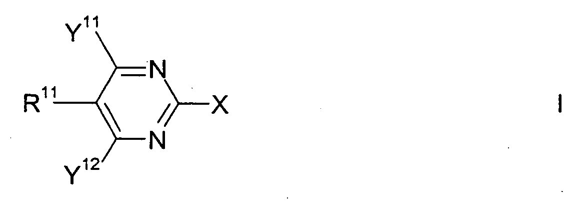 in which
R¹¹ is hydrogen, alkyl, alkoxy, oxaalkyl, thioalkyl, cyclohexyl, aryl, aralkyl or heterocyclyl;
X is Br or I; and
Y¹¹ and Y¹² are, independently of each other, hydrogen, F, CN, alkyl, alkoxy, oxaalkyl, thioalkyl, cyclohexyl, aryl, aralkyl or heterocyclyl;
**characterized in that**
a compound of general formula II 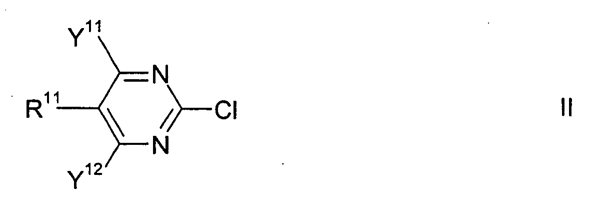 in which R¹¹, Y¹¹ and Y¹² are defined as in formula I above,
is reacted with a solution of HX, with X being Br or I, in an acid.

2. Method according to claim 1, **characterized in that** X is Br.

3. Method according to any one of claims 1 or 2, **characterized in that** both Y¹¹ and Y¹² are hydrogen.

4. Method according to any one of claims 1 to 3, **characterized in that** R¹¹ is alkyl or cycloalkyl.

5. Method according to claim 4, **characterized in that** R¹¹ is straight-chain alkyl.

6. Method according to any one of claims 1 to 5, **characterized in that** said acid is a carboxylic acid.

7. Method according to claim 6, **characterized in that** said carboxylic acid is non-aqueous and is selected from the group consisting of formic acid, acetic acid, propionic acid, and trifluoroacetic acid.

8. Method according to claim 7, **characterized in that** said non-aqueous carboxylic acid is glacial acetic acid.

9. Method according to any one of claims 1 to 8, **characterized in that** said reaction is performed at a temperature between about 20 °C and the reflux temperature of said acid.

10. Method according to any one of claims 1 to 9, **characterized in that** said reaction is performed in two steps (A) and (B), said steps being
(A) reacting the compound of formula II with HX dissolved in said acid at a temperature in the range of about 20 °C to about 35 °C; and
(B) refluxing said reaction mixture of step (A).
